# EUROPEAN PATENT APPLICATION

(11) **EP 4 846 876 A2**
(43) Date of publication of application: **02.12.2026**
(21) Application number: 26213004.0
(22) Date of filing: 07.12.2021
(51) Int. Cl.: A24F 40/70

(54) **ATOMIZATION CORE, ATOMIZER, AND ELECTRONIC ATOMIZATION DEVICE**

(62) Document number(s) of the earlier application(s) in accordance with Art. 76 EPC: 21966670.8 / 4 445 774
(71) Applicant: Hainan Moore Brothers Technology Co., Ltd., Hainan 571900 (CN)
(72) Inventor: SHEN, Qijie, Hainan, 571924 (CN); JIANG, Zhenlong, Hainan, 571924 (CN); XIAO, Congwen, Hainan, 571924 (CN); LV, Hongxia, Hainan, 571924 (CN); LUO, Hongliang, Hainan, 571924 (CN)
(74) Representative: Herrero & Asociados, S.L.

(57) **Abstract**

The present application provides an atomization core (20), a method for making the atomization core (20), an atomizer (1), and an electronic atomization device. The atomization core (20) includes a substrate (21) including a first surface (213) and a second surface (214) that are opposite to each other, a heating element (22) disposed on an atomization surface (211) of the substrate (21), a conductor lead (23), an electrode (24) disposed on the first surface (213), and a bonding pad (25) disposed on the second surface. The substrate (21) defines a through hole (212) extending from the second surface (214) to the first surface (213). The conductor lead (23) is disposed in the through hole (212), and is fixed to the substrate (21) to form an integral structure. Two ends (231, 232) of the conductor lead (23) are connected to the electrode (24) and the bonding pad (25), respectively.

## Description

### TECHNICAL FIELD

This application relates to the technical field of atomizers, and particularly relates to an atomization core, a method for making the atomization core, an atomizer, and an electronic atomization device.

### BACKGROUND

In the related art, an electronic atomization device mainly consists of an atomizer and a power supply assembly. An atomization core in the atomizer is a core component, and the atomization core mainly includes a ceramic atomization core and a liquid guide cotton atomization core. The traditional ceramic atomization core is obtained by forming a layer of heating film resistor on the surface of a ceramic substrate through screen printing and sintering and a group of conductor leads connected to a power supply through sintering attaching. However, the contact stability between the conductor leads and the ceramic substrate of the ceramic atomization core is poor, and damage may easily occur.

### SUMMARY

In view of this, this application provides an atomization core, a method for making the atomization core, an atomizer, and an electronic atomization device to solve the problems that the contact stability between a conductor lead and a substrate of a ceramic atomization core is poor, and damage may easily occur in the related art.

In order to solve the foregoing technical problems, a first technical solution is to provide an atomization core including: a substrate, a heating element, a conductor lead, an electrode, and a bonding pad. The substrate includes a first surface and a second surface that are opposite to each other, and defines a through hole extending from the second surface to the first surface. The first surface is an atomization surface, and the substrate is configured to guide an aerosol-forming material to the atomization surface. The heating element is disposed on the atomization surface and configured to heat and atomize the aerosol-forming material to form an aerosol. The conductor lead is disposed in the through hole and fixed to the substrate to form an integral structure. The electrode is disposed on the first surface and electrically connected to the heating element. The bonding pad is disposed on the second surface and configured to be connected to a power supply assembly. A first end of the conductor lead is electrically connected to the electrode, and a second end of the conductor lead is electrically connected to the bonding pad.

In some embodiments, the electrode, the bonding pad, and the conductor lead form another integral structure.

In some embodiments, the through hole is a straight-through hole perpendicular to the first surface.

In some embodiments, the electrode on the first surface at least covers a part of the through hole, and the bonding pad on the second surface at least covers another part of the through hole.

In some embodiments, the electrode and the bonding pad are both at least partially embedded in the substrate.

In some embodiments, the conductor lead is a solid conductor, or an interior of the conductor lead defines a pore.

In some embodiments, a solid part of the conductor lead accounts for more than 50% of a volume of the through hole.

In some embodiments, a lead diameter of the conductor lead ranges from 0.1 mm to 1 mm; and/or a material of the conductor lead includes one or more selected from the group consisting of Ag, Cu, and Au.

In some embodiments, the substrate is a porous substrate, and a porosity of the substrate ranges from 30% to 80%; and/or a pore diameter of the pore of the substrate ranges from 10 µm to 200 µm.

In order to solve the foregoing technical problems, a second technical solution is to provide a method for making an atomization core, including:
providing a substrate defining a through hole; wherein the substrate includes a first surface and a second surface that are opposite to each other, the through hole extends from the second surface to the first surface, the first surface is an atomization surface, and the substrate is configured to guide an aerosol-forming material to the atomization surface;
disposing a heating element and an electrode on the first surface, and electrically connecting the electrode to the heating element, wherein the heating element is configured to heat and atomize the aerosol-forming material to form an aerosol;
disposing a bonding pad on the second surface, wherein the bonding pad is configured to be connected to a power supply assembly; and
disposing a conductor lead in the through hole, electrically connecting a first end of the conductor lead to the electrode, and electrically connecting a second end of the conductor lead to the bonding pad.

In some embodiments, the disposing the conductor lead in the through hole includes:
filling a conductive paste into the through hole; and
sintering the conductive paste in the through hole to obtain the conductor lead.

In some embodiments, a side wall of the through hole defines a depression, and the disposing the conductor lead in the through hole includes:
filling the conductive paste into the through hole, and sintering the conductive paste in the through hole to obtain the conductor lead, wherein a protrusion is disposed on a side wall of the conductor lead, the protrusion is embedded in the depression, so that the conductor lead and the substrate are combined to form an inter-embedded structure with an uneven edge.

In some embodiments, a surface of the side wall of the through hole is in a smooth state, and the disposing the conductor lead in the through hole includes:
filling the conductive paste into the through hole, and sintering the conductive paste in the through hole to obtain the conductor lead, wherein a surface of the side wall of the conductor lead is in the smooth state.

In some embodiments, the disposing the conductor lead in the through hole, the electrically connecting the first end of the conductor lead to the electrode, and the electrically connecting the second end of the conductor lead to the bonding pad include:
filling a conductive paste into the through hole, disposing the electrode on the first surface, and disposing the bonding pad on the second surface; and
performing co-sintering on the conductive paste in the through hole, the electrode and the bonding pad, so that the conductor lead, the electrode, and the bonding pad form an integral structure.

In order to solve the foregoing technical problems, a third technical solution is to provide an atomizer, including a housing and an atomization core. The housing defines an accommodating cavity. The atomization core is disposed in the accommodating cavity and matched with the housing to form a liquid storage cavity. The atomization core is configured to heat and atomize an aerosol-forming material from the liquid storage cavity when powered on to form an aerosol. The atomization core is the atomization core of any one of the above embodiments in the first technical solution or an atomization core that is prepared by the method of any one of the above embodiments in the second technical solution.

In order to solve the foregoing technical problems, a fourth technical solution is to provide an electronic atomization device, including the atomizer in the third technical solution and a power supply assembly. The power supply assembly is electrically connected to the conductor lead of the atomizer and configured to supply power to the atomizer.

This application has the following beneficial effects. This application differs from the related art in that the atomization core of this application includes a substrate, a heating element and a conductor lead. The substrate has an atomization surface and a through hole extending to the atomization surface. The substrate is configured to guide aerosol-forming material to the atomization surface. The heating element is arranged on the atomization surface and is configured to heat and atomize the aerosol-forming material to form an aerosol. The conductor lead is arranged in the through hole and fixed to the substrate to form an integral structure. A first end of the conductor lead is electrically connected to the heating element, and a second end of the conductor lead is configured to connect a power supply assembly. In this application, the conductor lead is arranged in the substrate to form an integral structure with the substrate, so that the connection direction of the heating element and the power supply assembly is changed, the conductor lead is prevented from shielding the atomization surface, the problems that the contact stability between the conductor lead and the substrate of the ceramic atomization core is poor, and damage may easily occur are solved, and the atomization conversion efficiency is improved to a greatest extent.

### BRIEF DESCRIPTION OF THE DRAWINGS

To describe the technical solutions of the embodiments of this application more clearly, the following briefly introduces the accompanying drawings required for describing the embodiments. Apparently, the accompanying drawings in the following description show only some embodiments of this application, and a person of ordinary skill in the art may still derive other drawings from these accompanying drawings without creative efforts.
FIG. 1 is a schematic structural diagram of an electronic atomization device provided by this application.
FIG. 2 is a schematic structural diagram of an atomizer provided by this application.
FIG. 3 is a schematic structural diagram of an atomization core in an embodiment provided by this application.
FIG. 4 is a front-view schematic structural diagram of the atomization core provided in FIG. 3.
FIG. 5 is a bottom-view schematic structural diagram of the atomization core provided in FIG. 3.
FIG. 6 is a cross-sectional view of a first embodiment of the atomization core provided in FIG. 5 in Direction A-A.
FIG. 7 is a cross-sectional view of a second embodiment of the atomization core provided in FIG. 5 in Direction A-A.
FIG. 8 is a cross-sectional view of a third embodiment of the atomization core provided in FIG. 5 in Direction A-A.
FIG. 9 is a cross-sectional view of a fourth embodiment of the atomization core provided in FIG. 5 in Direction A-A.
FIG. 10 is a schematic structural diagram of an atomization core in another embodiment provided by this application.
FIG. 11 is a lateral cross-sectional view of connection between conductor lead and a substrate in an embodiment provided by this application.
FIG. 12 is a cross-sectional view of a structure of a first embodiment of the conductor lead provided by this application.
FIG. 13 is a cross-sectional view of a structure of a second embodiment of the conductor lead provided by this application.
FIG. 14 is a cross-sectional view of a structure of a third embodiment of the conductor lead provided by this application.
FIG. 15 is a cross-sectional view of a connection structure of the substrate and the conductor lead provided by this application.

### DETAILED DESCRIPTION

The technical solutions in embodiments of this application are clearly and completely described in the following with reference to the accompanying drawings in the embodiments of this application. Apparently, the described embodiments are merely some rather than all of the embodiments of this application. All other embodiments obtained by a person of ordinary skill in the art based on the embodiments of this application without creative efforts shall fall within the protection scope of this application.

The terms "first" and "second" in this application are merely intended for a purpose of description, and shall not be understood as indicating or implying relative significance or implicitly indicating the number of indicated technical features. Therefore, features defining "first" or "second" may explicitly or implicitly include at least one of the features. All directional indications (for example, up, down, left, right, front, back...) in the embodiments of this application are only used for explaining relative position relationships, movement situations, or the like between the various components in a specific posture (as shown in the accompanying drawings). If the specific posture changes, the directional indications change accordingly. In addition, the terms "include", "have", and any variant thereof are intended to cover a non-exclusive inclusion. For example, a process, method, system, product, or device that includes a series of steps or units is not limited to the listed steps or units; and instead, further optionally includes a step or unit that is not listed, or further optionally includes another step or unit that is intrinsic to the process, method, product, or device. Embodiment mentioned in the specification means that particular features, structures, or characteristics described with reference to the embodiment may be included in at least one embodiment of this application. The term appearing at different positions of this specification may not refer to the same embodiment or an independent or alternative embodiment that is mutually exclusive with another embodiment. A person skilled in the art explicitly or implicitly understands that the embodiments described in the specification may be combined with other embodiments.

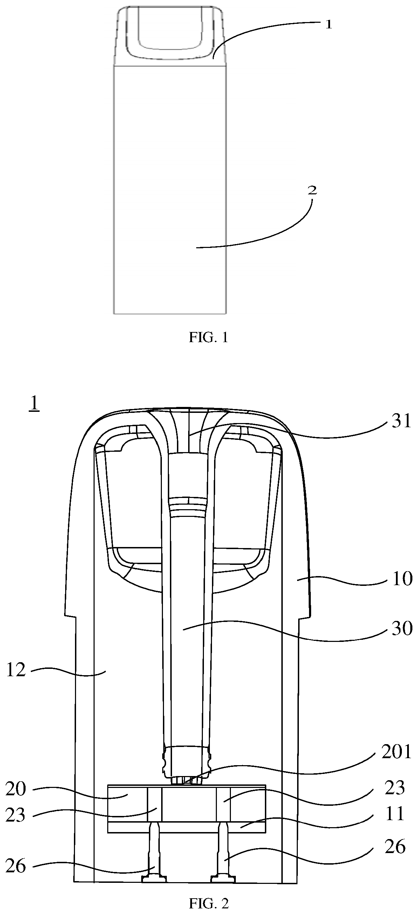

Referring to FIG. 1, FIG. 1 is a schematic structural diagram of an electronic atomization device provided by this application.

The electronic atomization device includes an atomizer 1 and a power supply assembly 2. The power supply assembly 2 is connected to the atomizer 1, and is configured to supply power to the atomizer 1. The electronic atomization device may be configured to atomize a liquid substrate. The atomizer 1 is configured to store a liquid aerosol-forming material and atomize the aerosol-forming material to form an aerosol capable of being inhaled by a user. The liquid aerosol-forming material may be a liquid substrate such as a medicinal liquid and a plant grass leaf type aerosol-forming material. The atomizer 1 may be specifically used in different fields, such as medical treatment, beauty treatment and recreational inhalation. The power supply assembly 2 includes a battery (not shown), an airflow sensor (not shown), and a controller (not shown). The battery is configured to supply power to the atomizer 1 and control the heating power, heating duration, etc. of an atomization core 20 so that the atomizer 1 may atomize aerosol-forming material to form an aerosol. The airflow sensor is configured to detect an airflow change in the electronic atomization device, and the controller starts the electronic atomization device according to the airflow change detected by the airflow sensor. The atomizer 1 and the power supply assembly 2 may be arranged integrally or may be detachably connected, which is designed according to specific requirements.

Referring to FIG. 2, FIG. 2 is a schematic structural diagram of an atomizer provided by this application.

The atomizer 1 includes a housing 10 and an atomization core 20. The housing 10 is provided with an accommodating cavity 11. The atomization core 20 and the housing 10 may be nondetachably connected in an integral arrangement manner, and may also be detachably connected. In this embodiment, the atomization core 20 and the housing 10 are detachably connected, and the atomization core 20 is directly connected to the housing 10, so that the atomization core 20 and the housing 10 may be detachably connected without introducing an additional conduit, the volume of the atomizer 1 is reduced, and the use is more convenient. It can be understood that the atomizer 1 of this application is a portable atomizer. The atomization core 20 is arranged in the accommodating cavity 11, is matched with the housing 10 to form a liquid storage cavity 12, and is configured to store aerosol-forming material. The atomization core 20 may be used in different fields, such as medicine atomization and oil flower and grass liquid atomization, and is configured to heat and atomize the aerosol-forming material from the liquid storage cavity 12 when powered on to form an aerosol. The atomizer 1 may further include an installing seat (not shown in the figures), and the installing seat is configured to install the atomization core 20.

Specifically, protrusions (not shown in the figures) are formed on the outer wall surface of the atomization core 20, chutes (not shown in the figures) are formed on the outer wall surface of the housing 10, and limiting blocks (not shown in the figures) are arranged in the chutes. The protrusions on the atomization core 20 are aligned with and inserted into the chutes of the housing 10, the atomization core 20 or the housing 10 is rotated so that the protrusions are limited by the limiting blocks in the chutes, the fixation of the atomization core 20 to the housing 10 is realized, and the detachable connection between the atomization core 20 and the housing 10 is realized. It can be understood that protrusions may also be formed on the outer wall surface of the housing 10, chutes may be formed on the outer wall surface of the atomization core 20, limiting blocks may be arranged in the chutes, and the detachable connection between the atomization core 20 and the housing 10 is realized. The detachable connection between the atomization core 20 and the housing 10 may also be realized by using a magnetic suction manner. The specific implementation is not limited as long as the detachable connection between the atomization core 20 and the housing 10 is realized.

In an embodiment, the atomization surface of the atomization core 20 faces an upward direction, and the atomization amount may be improved. When the atomization surface faces the upward direction, a pin (not shown in the figures) of the atomization core 20 may be arranged in any one position of the atomization core 20. In this embodiment, the pin is arranged downwards, and the atomizer 1 may be conveniently and automatically assembled. An inhaling passage 30 is arranged on one side of the atomization core 20 far away from the power supply assembly 2, and the inhaling passage 30 communicates with an atomization cavity 201. An inhaling opening 31 at one side of the inhaling passage 30 far away from the power supply assembly 2 communicates with the atmosphere, so that the aerosol in the atomization cavity 201 may flow out through the inhaling passage 30 and may be provided for a user to be inhaled from the inhaling opening 31.

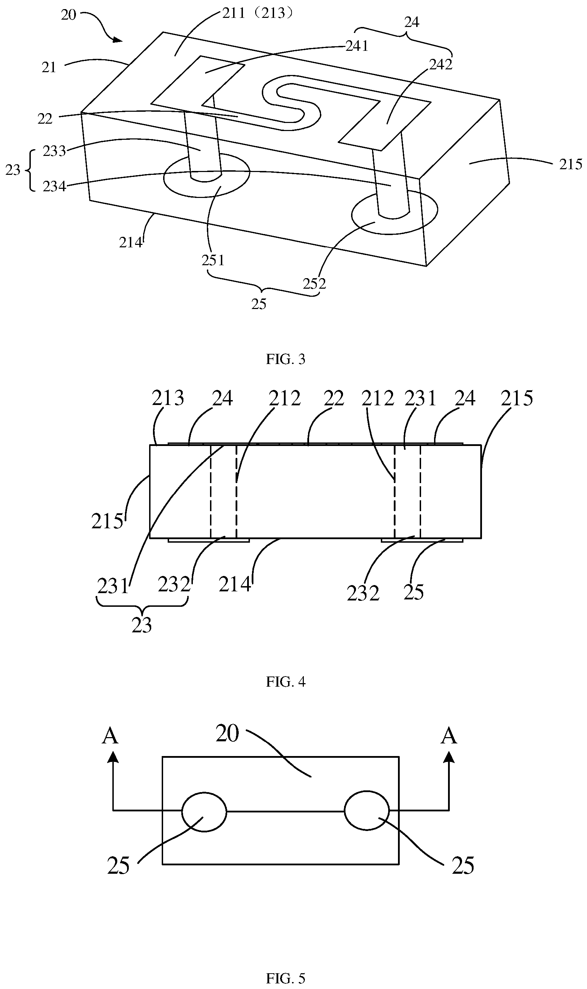

Referring to FIG. 3 and FIG. 4, FIG. 3 is a schematic structural diagram of an atomization core in an embodiment provided by this application, and FIG. 4 is a front-view schematic structural diagram of the atomization core provided in FIG. 3.

In an embodiment, the atomization core 20 includes a substrate 21, a heating element 22 and a conductor lead 23.

Specifically, the substrate 21 may be a porous substrate or a perforated compact substrate. The porous substrate concretely may be a porous ceramic substrate, and the perforated compact substrate may be a perforated glass substrate or a compact ceramic substrate, etc. The substrate 21 in this embodiment is made of porous ceramic. The porous ceramic material is generally a ceramic material obtained by treating components such as an aggregate, a bonding agent and a pore former through high-temperature sintering. A great number of pore structures which communicate with each other and communicate with the surface of the material are formed inside the porous ceramic material. Due to its good performance such as high porosity, stable chemical properties, large specific surface area, small volume density, low heat conductivity, high-temperature resistance and corrosion resistance, the porous ceramic material has wide application in the fields of metallurgy, biology, energy source, environment protection, etc.

The substrate 21 may be in a flat plate shape or a step shape, and it is not specifically limited by this application. The substrate 21 has a first surface 213 and a second surface 214, the first surface 213 is the surface of the substrate 21 at one side facing the liquid storage cavity 12, and the second surface 214 is the surface of the substrate 21 at one side facing away from the first surface 213. Both the first surface 213 and the second surface 214 may be flat planes, the first surface 213 and the second surface 214 may also be irregular surfaces such as curve surfaces, and they are not specifically limited by this application. For example, a groove (not shown in the figures) is formed in one side of the first surface 213 of the substrate 21, and the surface of the groove also belongs to the first surface 213.

The substrate 21 has an atomization surface 211 and a through hole 212 extending to the atomization surface 211, and is configured to guide aerosol-forming material to the atomization surface 211. In this embodiment, the porosity of the substrate 21 ranges from 30% to 80%, and/or the pore diameter of the pores of the substrate 21 ranges from 10 µm to 200 µm. It can be understood that the higher the porosity of the substrate 21 is, the higher the liquid guide speed is. Meanwhile, the pore diameter of the pores of the substrate 21 is associated with protrusions of the conductor lead 23. Due to this range, the matching rate of the conductor lead 23 and the substrate 21 may be the highest. Meanwhile, the conduction of the conductor lead 23 and the flow guide on the aerosol-forming material by the substrate 21 may be convenient. In this embodiment, the hole diameter of the through hole 212 ranges from 0.1 mm to 1 mm. In other embodiments, the hole diameter of the through hole 212 and the porosity of the substrate 21 may be set according to requirements, and it is not limited in this application.

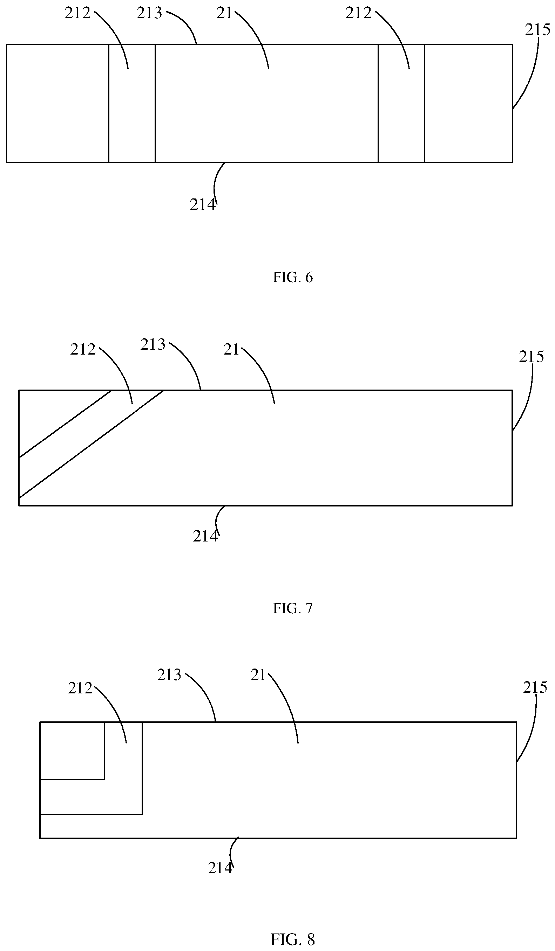

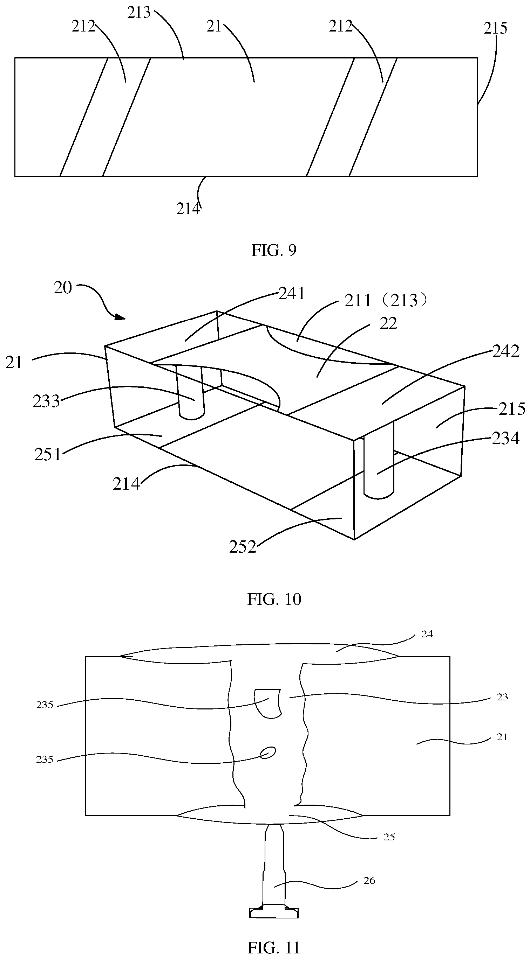

Referring to FIG. 5 to FIG. 8, FIG. 5 is a bottom-view schematic structural diagram of the atomization core provided in FIG. 3. FIG. 6 is a cross-sectional view of a first embodiment of the atomization core provided in FIG. 5 in Direction A-A. FIG. 7 is a cross-sectional view of a second embodiment of the atomization core provided in FIG. 5 in Direction A-A. FIG. 8 is a cross-sectional view of a third embodiment of the atomization core provided in FIG. 5 in Direction A-A. FIG. 9 is a cross-sectional view of a fourth embodiment of the atomization core provided in FIG. 5 in Direction A-A.

In an embodiment, the substrate 21 has a first surface 213, a second surface 214 and a side surface 215. The second surface 214 and the first surface 213 are arranged facing away from each other, and the side surface 215 is connected to the first surface 213 and the second surface 214. Generally, the first surface 213 may be configured to be in contact with the aerosol-forming material communicating with the liquid storage cavity 12, the second surface 214 may be configured to be in contact with gas. The contact with gas mentioned herein may refer to that the second surface 214 is in contact with external air, in contact with air in the atomization cavity 201, or in contact with the air in the inhaling passage 30, etc.

In this embodiment, the aerosol-forming material located at one side of the second surface 214 of the substrate 21 penetrates to the side where the first surface 213 of the substrate 21 is located through a great number of pore structures which communicate with each other and communicate with the surface of the material inside the substrate 21, the heating element 22 is arranged on the first surface 213 to atomize the aerosol-forming material penetrating to the first surface 213. Pore structures also communicate with the side surface 215. Therefore, the side surface 215 may also be used for liquid guide or ventilation.

As shown in FIG. 3 and FIG. 4, in the first embodiment, the substrate 21 has the first surface 213 and the second surface 214 in opposite arrangement, and the first surface 213 is the atomization surface 211. The through hole 212 extends from the first surface 213 to the second surface 214, and liquid may be conveniently sucked from the side surface 215 for forming an aerosol. Meanwhile, liquid may be sucked from the second surface 214 for forming an aerosol.

In the first embodiment, the through hole 212 is a straight-through hole perpendicular to the first surface 213, so that the through hole may be conveniently prepared. If the through hole 212 is the straight-through hole perpendicular to the first surface 213, meanwhile, a raw material of the substrate may be perforated to form multiple through holes in one step, and may be cut into a plurality of substrates 21. Meanwhile, mold preparation may also be convenient, and the efficiency is high.

In the second embodiment, the substrate 21 also has the first surface 213 and the second surface 214 in opposite arrangement, and the side surface 215 connected to the first surface 213 and the second surface 214. The first surface 213 is the atomization surface 211, and the through hole 212 may extend from the first surface 213 to the side surface 215. Specifically, as shown in FIG. 7, the through hole 212 may be an inclined hole structure extending to the side surface 215. That is, the through hole 212 is a straight hole facing the side surface 215.

As shown in FIG. 8, in the third embodiment, the through hole 212 may also be a cornered through hole connected to the side surface 215 and the first surface 213. When the through hole 212 is perforated through extending from the first surface 213 to the side surface 215, mold stripping is difficult, meanwhile, the mold cannot be perforated to form multiple through holes, and the perforation efficiency is low. However, the through hole 212 is of a structure extending from the first surface 213 to the side surface 215, liquid suction from the second surface 214 may be realized, and such a structure is suitable for a downward-atomization atomizer. The structure may be set according to requirements in practical use, and it is not limited in this application.

As shown in FIG. 9, in the fourth embodiment, the through hole 212 may further be a straight-through hole/non-straight-through hole forming a certain oblique angle with the first surface 213 or the side surface 215. When the through hole 212 is the straight-through hole/non-straight-through hole forming a certain oblique angle, identically, mold stripping is difficult, meanwhile, the mold cannot be perforated to form multiple through holes, and the perforation efficiency is low. However, liquid suction from the second surface 214 may be realized, and such a structure is suitable for a downward-atomization atomizer. The structure may be specifically set according to requirements. It can be understood that the quantity of the through hole(s) 212 may be one or more, and when the quantity of the through holes 212 is more, the extending direction of the through holes 212 may be parallel or non-parallel to each other, they are not limited in this application as long as the quantity of the through holes 212 is matched with the quantity of the conductor leads 23.

As shown in FIG. 3 and FIG. 4, the heating element 22 is arranged on the atomization surface 211, and the heating element 22 is configured to atomize aerosol-forming material guided out through the substrate 21. The atomization surface 211 absorbs heat of the heating element 22, and is thus configured to heat and atomize aerosol-forming material when powered on to form an aerosol. In this embodiment, the heating element 22 uses a metal heating film which has a good heat conduction effect. In other embodiments, the heating element 22 may further be at least one of a heating coating, a heating line, a heating sheet or a heating net, and it is not limited in this application.

In this embodiment, a porous ceramic material is used for making the substrate 21, the aerosol-forming material located at one side of the substrate 21 penetrates to the other side of the substrate 21 through a great number of pore structures which are inside the porous ceramic material and communicate with each other and communicate with the material surface, and is additionally in contact with the heating element 22 arranged on one side surface of the substrate 21, so that the aerosol-forming material is atomized into the aerosol.

As shown in FIG. 3, in an embodiment, the heating element 22 is in an S shape, the heating element 22 may be of an integrally formed structure or a detachable structure, and it may be set according to requirements. In this embodiment, the thickness of the metal heating film used by the heating element 22 ranges from 50 µm to 120 µm, both the heating efficiency and the heat conductivity of the heating element 22 are high, and the atomization efficiency of the aerosol-forming material can be improved. In other embodiments, the heating element 22 may further be in a rectangular shape, an oval shape or a round shape, etc., additionally, the thickness, size, quantity, etc. of the heating element 22 may be set according to requirements, and they are not limited in this application.

As shown in FIG. 3, in an embodiment, the atomization core 20 further includes an electrode 24 and a bonding pad 25. The electrode 24 is arranged on the first surface 213 and is electrically connected to the heating element 22. The bonding pad 25 is arranged on the second surface 214 and is configured to be connected to the power supply assembly 2.

Specifically, two electrodes 24 are used and include a first electrode 241 and a second electrode 242, and the first electrode 241 and the second electrode 242 are arranged at interval and are both connected to the heating element 22. The heating element 22 is configured to atomize the aerosol-forming material guided out through the substrate 21. Specifically, the heating element 22 may further be at least one of a heating coating, a heating line, a heating sheet or a heating net, and the heating element 22 is electrically connected to the power supply assembly 2 through the two electrodes 24. The first electrode 241 and the second electrode 242 may be arranged on a part of region on the atomization surface 211, and may extend to the edge of the atomization surface 211, and it is not limited in this application.

Specifically, as shown in FIG. 3, the quantity of the bonding pad(s) 25 may be one or more, the bonding pads may be set to be in a shape of a cylinder or a cuboid, and may be set specifically according to requirements as long as the quantity of the bonding pads 25 is matched with the quantity of the conductor leads 23. One end of the conductor lead 23 is connected to the electrode 24, and the other end of the conductor lead is connected to the bonding pad 25. In this embodiment, two bonding pads 25 are used and include a first bonding pad 251 and a second bonding pad 252, and the first bonding pad 251 and the second bonding pad 252 are arranged at interval. The first bonding pad 251 and the second bonding pad 252 are respectively connected to two conductor leads 23, and the two conductor leads 23 vertically penetrate through the two electrodes 24 and the two bonding pads 25. Specifically, the two conductor leads 23 include a first conductor lead 233 and a second conductor lead 234, the first conductor lead 233 vertically penetrates through the first electrode 241 and the first bonding pad 251, the second conductor lead 234 vertically penetrates through the second electrode 242 and the second bonding pad 252, and the two ends of the first conductor lead 233 are electrically connected to the first electrode 241 and the first bonding pad 251 respectively, the two ends of the second conductor lead 234 are electrically connected to the second electrode 242 and the second bonding pad 252 respectively. In other embodiments, the bonding pads 25 may further be arranged on the side surface 215, and the bending of the conductor leads 23 and the liquid suction shield of the second surface 214 may be avoided by adopting this arrangement manner. Therefore, the specific position of the bonding pads 25 may be set according to specific requirements, and it is not limited in this application.

Referring to FIG. 10, FIG. 10 is a schematic structural diagram of an atomization core in another embodiment provided by this application.

As shown in FIG. 10, in another embodiment, the heating element 22 is in a waist shape with two wide ends and the gradually decreased middle, the two ends of the heating element 22 are respectively connected to the first electrode 241 and the second electrode 242, and the first electrode 241 and the second electrode 242 are arranged at interval, and are connected to the two ends of the waist-shaped heating element 22 respectively. Additionally, the first bonding pad 251 and the second bonding pad 252 are arranged on the second surface 214 of the substrate 21, and the first bonding pad 251 and the second bonding pad 252 are arranged at interval. The first conductor lead 233 is connected to the first electrode 241 and the first bonding pad 251, and the second conductor lead 234 is connected to the second electrode 242 and the second bonding pad 252. In this embodiment, the thickness of the metal heating film used by the waist-shaped heating element 22 ranges from 10 µm to 50 µm, both the heating efficiency and the heat conductivity of the waist-shaped heating element 22 are high, and the atomization efficiency of the aerosol-forming material can be improved.

Optionally, a projection of the first electrode 241 on the first surface 213 or the second surface 214 and a projection of the first bonding pad 251 on the first surface 213 or the second surface 214 may overlap with each other, and the size of the projection of the first electrode 241 is the same as the size of the projection of the first bonding pad 251, and the first electrode 241 and the first bonding pad 251 may further be the electrode and the bonding pad with different sizes. In some embodiments, the structure of the projection of the first electrode 241 is the same as the structure of the projection of the first bonding pad 251, and an arrangement with the laminated projection is adopted, so that the region corresponding to the first electrode 241 and the region corresponding to the first bonding pad 251 may be printed through the same printing screen, so the preparation is convenient.

As shown in FIG. 3, FIG. 4 and FIG. 10, in some embodiments, the conductor lead 23 is arranged in the through hole 212. The conductor lead 23 includes a first end 231 and a second end 232, the first end 231 is electrically connected to the electrode 24, and the second end 232 is configured to connect the power supply assembly 2. Specifically, the first end 231 of the conductor lead 23 is electrically connected to the electrode 24, and the second end 232 is electrically connected to the bonding pad 25.

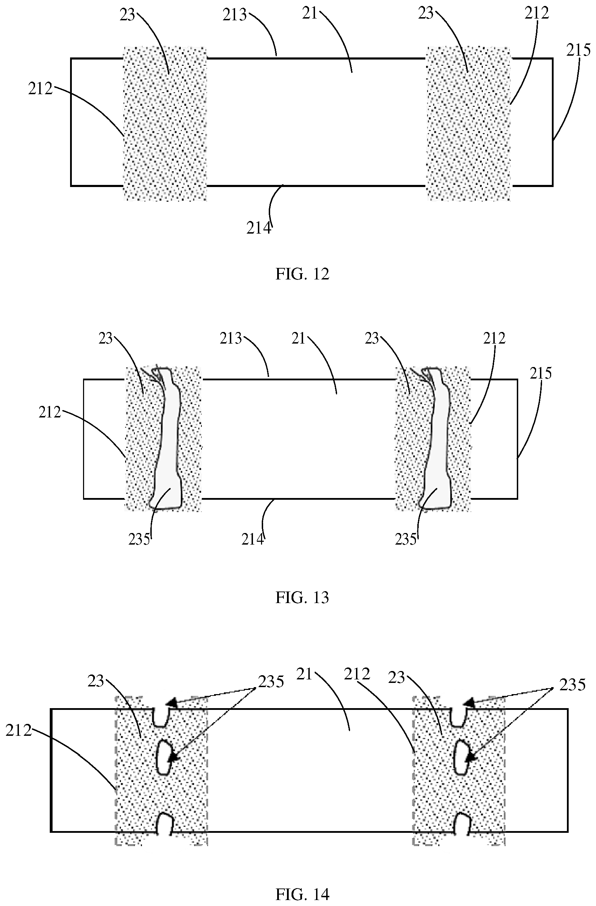

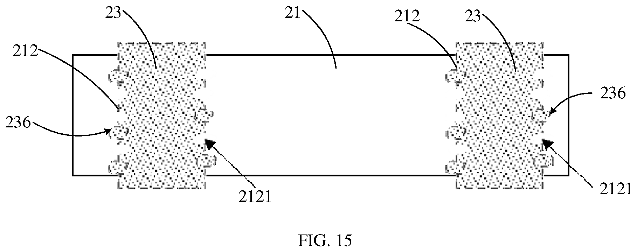

Referring to FIG. 11 to FIG. 15, FIG. 11 is a lateral cross-sectional view of connection between a conductor lead and a substrate in an embodiment provided by this application. FIG. 12 is a cross-sectional view of a structure of the first embodiment of the conductor lead provided by this application. FIG. 13 is a cross-sectional view of a structure of the second embodiment of the conductor lead provided by this application. FIG. 14 is a cross-sectional view of a structure of the third embodiment of the conductor lead provided by this application. FIG. 15 is a cross-sectional view of a connection structure of the substrate and the conductor lead provided by this application.

As shown in FIG. 11 to FIG. 15, in an embodiment, the conductor lead 23, the electrode 24 and the bonding pad 25 are all arranged in the through hole 212, and are all prepared by a method of filling conductive paste into the through hole 212 and then performing sintering. Optionally, the lead diameter of the conductor lead 23 ranges from 0.1 mm to 1 mm. A material used by the conductor lead 23 is one or more of Ag, Cu and Au, and may be specifically designed according to requirements, and it is not limited in this application.

Specifically, in this embodiment, a metal or alloy material with the highest conductibility, such as one or a combination of more of Ag, Cu, Au, etc. is used, paste form metal of the metal or the alloy material is screen-printed and filled into the through hole 212 to be matched and co-sintered with the porous structure ceramic substrate 21 to form an integral structure of the conductor lead 23, the electrode 24, the bonding pad 25 and the substrate 21. The integral structure may be a nondetachable structure. Specifically, the integral structure is not a structure which is perforated for inserting leads, and is also not a structure with the clamped substrate 21 and conductor lead 23.

Meanwhile, the integral structure is different from an integrally formed structure, and is an indivisible and nondetachable structure formed by the substrate 21 and the paste form of the metal or alloy material through co-sintering.

The conductor lead 23 after matching and co-sintering has the following characteristics. The conductor lead 23 vertically penetrates through and is connected to the electrode 24 and the bonding pad 25, and the direct current resistance of the conductor lead 23 is less than 0.1 Ω. The lead diameter of the conductor lead 23 ranges from 0.1 mm to 1 mm. The solid part of the conductor lead 23 accounts for more than 50% of the volume of the through hole 212. That is, the conductor lead 23 may be hollow or solid conductor, but the lowest filling rate is 50%. Specifically, the interior of the conductor lead 23 may be of a compact structure, as shown in FIG. 12. The interior of the conductor lead 23 may also have pores 235, and the pores 235 are bubbles naturally formed in a process of screen-printing and filling the paste form metal of the metal or the alloy material into the through hole 212 to form the conductor lead 23. The conductor lead 23 and the substrate 21 of the porous ceramic structure are matched and co-sintered to form an integral structure, the formed conductor lead 23 and the through hole 212 of the substrate 21 are in a co-sintered embedding state, dismounting or falling cannot occur after the co-sintering and shaping, and the reliability of the conductor lead 23 is greatly improved. This application is different from a structure that the lead is arranged outside the substrate 21 or partially arranged inside the substrate 21 and partially arranged outside the substrate 21 in the related art, and the problem that the conductor lead 23 may easily break off or be damaged through being pulled in the production and assembly process is solved.

The pores 235 may be through holes or blind holes, as shown in FIG. 13, the pores 235 may be in irregular pore shapes in the conductor lead 23. As shown in FIG. 14, the pores 235 may exist on the surface of the conductor lead 23, may exist inside the conductor lead 23, and are bubbles naturally formed by screen-printing and filling the paste form metal of the metal or alloy material into the through hole 212 to be sintered, the shapes and sizes of the pores 235 may be in any states, and they are not limited in this application.

As shown in FIG. 15, the substrate 21 is of a porous structure, so that the side wall of the through hole 212 formed inside the substrate are in an uneven state. In an embodiment, the conductor lead 23 has protrusions 236 on the side wall, the through hole 212 has depressions 2121 on the side wall, and the protrusions 236 are embedded in the depressions 2121, so that the edge of the conductor lead 23 combined with the substrate 21 is uneven rough edge, the combination is firmer, the conductor lead 23 is prevented from falling off from the substrate 21, and the stability is high. In other embodiments, the side wall of the conductor lead 23 and the side wall of the through hole 212 may also be in a smooth state, and it is not limited in this application.

In this embodiment, as shown in FIG. 2, FIG. 3 and FIG. 11, an ejector pin 26 in direct contact with the bonding pad 25 is arranged at the bottom of each bonding pad 25, and is configured to conduct the heating element 22 and the power supply assembly 2. During working, the ejector pin 26 has a longitudinal stress direction, i.e., the direction from the first surface 213 to the second surface 214. When the ejector pin 26 exerts acting force, a mutual embedding structure of the substrate 21 and the conductor lead 23 may achieve a limiting effect, the conduction contact stability between the substrate and the conductor lead is enhanced, meanwhile, the mechanical property is excellent, the conductor lead 23 is prevented from falling off from the substrate 21, and the conduction is more stable.

The atomization core disclosed by this application includes a substrate, a heating element and a conductor lead. The substrate has an atomization surface and a through hole extending to the atomization surface. The substrate is configured to guide aerosol-forming material to the atomization surface. The heating element is arranged on the atomization surface and is configured to heat and atomize the aerosol-forming material to form an aerosol. The conductor lead is arranged in the through hole and fixed to the substrate to form an integral structure. A first end of the conductor lead is electrically connected to the heating element, and a second end of the conductor lead is configured to connect a power supply assembly. In this application, the conductor lead is arranged in the substrate to form an integral structure with the substrate, so that the connection direction of the heating element and the power supply assembly is changed, the conductor lead is prevented from shielding the atomization surface, the problems that the contact stability between the conductor lead and the substrate of the ceramic atomization core is poor, and damage may easily occur are solved, and the atomization conversion efficiency is improved to a greatest extent.

The foregoing descriptions are merely implementations of this application, and the patent scope of this application is not limited thereto. All equivalent structure or equivalent process changes made according to the content of this specification and accompanying drawings in this application or by directly or indirectly applying this application in other related technical fields shall fall within the protection scope of this application.

## Claims

1. An atomization core (20), **characterized by** comprising:
a substrate (21), comprising a first surface (213) and a second surface (214) that are opposite to each other, and defining a through hole (212) extending from the second surface (214) to the first surface (213), wherein the first surface (213) is an atomization surface (211), and the substrate (21) is configured to guide an aerosol-forming material to the atomization surface (211);
a heating element (22), disposed on the atomization surface (211) and configured to heat and atomize the aerosol-forming material to form an aerosol;
a conductor lead (23), disposed in the through hole (212) and fixed to the substrate (21) to form an integral structure;
an electrode (24), disposed on the first surface (213) and electrically connected to the heating element (22); and
a bonding pad (25), disposed on the second surface (214) and configured to be connected to a power supply assembly (2);
wherein a first end (231) of the conductor lead (23) is electrically connected to the electrode (24), and a second end (232) of the conductor lead (23) is electrically connected to the bonding pad (25).

2. The atomization core (20) according to claim 1, wherein the electrode (24), the bonding pad (25), and the conductor lead (23) form another integral structure.

3. The atomization core (20) according to claim 1 or 2, wherein the through hole (212) is a straight-through hole perpendicular to the first surface (213).

4. The atomization core (20) according to any one of claims 1 to 3, wherein the electrode (24) on the first surface (213) at least covers a part of the through hole (212), and the bonding pad (25) on the second surface (214) at least covers another part of the through hole (212).

5. The atomization core (20) according to any one of claims 1 to 4, wherein the electrode (24) and the bonding pad (25) are both at least partially embedded in the substrate (21).

6. The atomization core (20) according to any one of claims 1 to 5, wherein the conductor lead (23) is a solid conductor, or an interior of the conductor lead (23) defines a pore.

7. The atomization core (20) according to any one of claims 1 to 6, wherein a solid part of the conductor lead (23) accounts for more than 50% of a volume of the through hole (212).

8. The atomization core (20) according to any one of claims 1 to 7, wherein a lead diameter of the conductor lead (23) ranges from 0.1 mm to 1 mm; and/or
a material of the conductor lead (23) comprises one or more selected from the group consisting of Ag, Cu, and Au.

9. The atomization core (20) according to any one of claims 1 to 8, wherein the substrate (21) is a porous substrate, and a porosity of the substrate (21) ranges from 30% to 80%; and/or
a pore diameter of the pore of the substrate (21) ranges from 10 µm to 200 µm.

10. A method for making an atomization core (20), comprising:
providing a substrate (21) defining a through hole (212); wherein the substrate (21) comprises a first surface (213) and a second surface (214) that are opposite to each other, the through hole (212) extends from the second surface (214) to the first surface (213), the first surface (213) is an atomization surface (211), and the substrate (21) is configured to guide an aerosol-forming material to the atomization surface (211);
disposing a heating element (22) and an electrode (24) on the first surface (213), and electrically connecting the electrode (24) to the heating element (22), wherein the heating element (22) is configured to heat and atomize the aerosol-forming material to form an aerosol;
disposing a bonding pad (25) on the second surface (214), wherein the bonding pad (25) is configured to be connected to a power supply assembly (2); and
disposing a conductor lead (23) in the through hole (212), electrically connecting a first end (231) of the conductor lead (23) to the electrode (24), and electrically connecting a second end (232) of the conductor lead (23) to the bonding pad (25).

11. The method for making the atomization core (20) according to claim 10, wherein the disposing the conductor lead (23) in the through hole (212) comprises:
filling a conductive paste into the through hole (212); and
sintering the conductive paste in the through hole (212) to obtain the conductor lead (23).

12. The method for making the atomization core (20) according to claim 11, wherein
a side wall of the through hole (212) defines a depression (2121), and the disposing the conductor lead (23) in the through hole (212) comprises:
filling the conductive paste into the through hole (212), and sintering the conductive paste in the through hole (212) to obtain the conductor lead (23), wherein a protrusion (236) is disposed on a side wall of the conductor lead (23), the protrusion (236) is embedded in the depression (2121), so that the conductor lead (23) and the substrate (21) are combined to form an inter-embedded structure with an uneven edge; or
a surface of the side wall of the through hole (212) is in a smooth state, and the disposing the conductor lead (23) in the through hole (212) comprises:
filling the conductive paste into the through hole (212), and sintering the conductive paste in the through hole (212) to obtain the conductor lead (23), wherein a surface of the side wall of the conductor lead (23) is in the smooth state.

13. The method for making the atomization core (20) according to claim 10, wherein the disposing the conductor lead (23) in the through hole (212), the electrically connecting the first end (231) of the conductor lead (23) to the electrode (24), and the electrically connecting the second end (232) of the conductor lead (23) to the bonding pad (25) comprise:
filling a conductive paste into the through hole (212), disposing the electrode (24) on the first surface (213), and disposing the bonding pad (25) on the second surface (214); and
performing co-sintering on the conductive paste in the through hole (212), the electrode (24) and the bonding pad (25), so that the conductor lead (23), the electrode (24), and the bonding pad (25) form an integral structure.

14. An atomizer (1), **characterized by** comprising:
a housing (10), defining an accommodating cavity (11); and
an atomization core (20), disposed in the accommodating cavity (11), and matched with the housing (10) to form a liquid storage cavity (12); wherein the atomization core (20) is configured to heat and atomize an aerosol-forming material from the liquid storage cavity (12) when powered on to form an aerosol, and the atomization core (20) is the atomization core according to any one of claims 1 to 9 or an atomization core that is prepared by the method according to any one of claims 10 to 13.

15. An electronic atomization device, **characterized by** comprising:
the atomizer (1) according to claim 14; and
a power supply assembly (2), electrically connected to the conductor lead (23) of the atomizer (1) and configured to supply power to the atomizer (1).
